# EUROPEAN PATENT APPLICATION

(11) **EP 2 698 070 A1**
(43) Date of publication of application: **19.02.2014**
(21) Application number: 13180786.9
(22) Date of filing: 16.08.2013
(51) Int. Cl.: A24F 47/00

(54) **An electronic cigarette structure**

(30) Priority: 17.08.2012 CN 201220408622 U
(71) Applicant: Shenzhen City Yukang Technology Co., Ltd., 518104 Shenzhen, Guangdong (CN)
(72) Inventor: Zhanghua, Yuan, 518104 Shenzhen Guangdong (CN)
(74) Representative: Alton, Andrew

(57) **Abstract**

An electronic cigarette structure is described and relates to the field of electronic cigarette technology. The electronic cigarette structure includes a round tubular cigarette tube and a control board module, a battery, an atomizer base, a fibreglass tube and a sealing ring installed within said cigarette tube from front to back. A diametrical hole penetrates the fibre glass tube, that hole being internally equipped with a glass fibre rope around which heating wire is wrapped, the heating wire being connected to the atomizer base, there being a cigarette mouthpiece installed at the rear end of the cigarette tube on the outside of the sealing ring, said mouthpiece being an acetate fibre filter cotton mouthpiece.

## Description

This invention relates to an electronic cigarette structure.

As people are paying more attention to health, they have become aware of the health hazards of tobacco, and this has led to the creation of electronic cigarettes. Electronic cigarettes, also known as virtual cigarettes and electronic nebulizers, have the same appearance as cigarettes and a similar taste to cigarettes and even have more flavour than ordinary cigarettes. It is also possible to inhale smoke and flavour in the same way as with regular cigarettes. Electronic cigarettes usually provide smoke to users by passing a tobacco liquid through an atomizer. Hazardous substances such as nicotine and tar can be extracted during the tobacco liquid production process, eliminating the dangers that these represent. Because the smoke contains no nicotine, tar or other harmful substances, after people smoke electronic cigarettes, their addiction to smoking will gradually decrease. As such, smoking electronic cigarettes can facilitate giving up smoking.

In existing electronic cigarettes, there is a hole located in the cigarette mouthpiece. When using an electronic cigarette, the smoke is sucked out through the hole in the middle of the cigarette mouthpiece. When a smoker uses excessive force or when there is excess tar, tar can easily be sucked into the mouth through the hole in the mouthpiece, causing an unpleasant taste in the mouth. Furthermore, existing electronic cigarettes all use mouthpieces constructed from silicone materials. From the point of view of smokers who habitually bite on cigarettes as they smoke, they find biting on hard cigarette mouthpieces strange. In addition to this, in the case of mouthpieces constructed from silicone materials, which all possess mouthpiece covers, when a smoker who habitually bites their cigarettes bites forcefully on such a mouthpiece, there is the possibility that the mouthpiece cover can pop into the smoker's mouth. So for those smokers who habitually bite on the cigarette whilst they are smoking, current electronic cigarettes exhibit certain hidden dangers in terms of their safety.

The purpose of this invention is to overcome the deficiencies of existing technologies, providing a kind of electronic cigarette with a cotton filter mouthpiece containing acetate fibre, which can be used more safely by smokers who habitually bite on cigarettes whilst they are smoking, whilst also preventing the possibility of tar being sucked directly into the mouth.

A first aspect of the invention provides an electronic cigarette including a round tubular cigarette tube and a control board module, a battery, an atomizer base, a fibreglass tube and a sealing ring installed within said tubular pipe from front to back, the area affected by improvement being: a diametrical hole penetrates said fibre glass tube, that hole being internally equipped with glass fibre rope around which heating wire is wrapped, the heating wire being connected to the atomizer base, there being a cigarette mouthpiece installed at the rear end of the cigarette tube on the outside of the sealing ring, said mouthpiece being an acetate fibre filter cotton mouthpiece.

The acetate fibres can be cellulose acetate fibres.

The mouthpiece can be a cotton-like filter mouthpiece containing or made of acetate fibre.

The mouthpiece can include or be a filter containing or made of acetate fibre and having the appearance and/or properties of cotton.

The front end of the aforementioned cigarette tube can be covered by a lamp cover, the lamp cover being equipped with an indicator light, said indicator light being connected to the control board module and battery and being controlled by the control board module.

The aforementioned hole in the fibreglass tube can be surrounded by a layer of non-woven cotton fabric.

The aforementioned non-woven cotton fabric can be surrounded by a layer of oil-absorbing cotton.

Said cigarette tube can be surrounded by a layer of adhesive paper.

The invention has a number of beneficial effects. Firstly, the invention uses an acetate fibre filter cotton mouthpiece, whereas those used in the current technologies are made of silicone material, so this invention looks more like a real cigarette. Secondly, the invention solves the problem of electronic cigarettes not being suitable for cigarette smokers who habitually bite on cigarette mouthpieces, ensuring that smoking this electronic cigarette brings almost the same feeling as smoking a real cigarette. Thirdly, as the mouthpiece is made of acetate fibre filter cotton, even if the smoker applies excessive force when drawing on the cigarette or there is excess tar, smoke can still be sucked out of the mouthpiece section, thus eliminating the possibility of tar being sucked directly into the mouth. Fourthly, for cigarette smokers who habitually bite cigarettes, this electronic cigarette gives almost the same feeling as smoking a real cigarette and when compared to existing electronic cigarettes, the situation where the mouthpiece cover pops into the smoker's mouth does not exist, so usage is more safe and reliable.

An embodiment of the invention will now be described in detail, by way of example only, and with reference to the accompanying drawings, in which:
Figure 1 shows an exploded schematic diagram of the overall structure of the present invention; and
Figure 2 shows a schematic view of the assembled invention.

The following when taken in conjunction with the appended diagrams and embodiments provides a more detailed description of this invention.

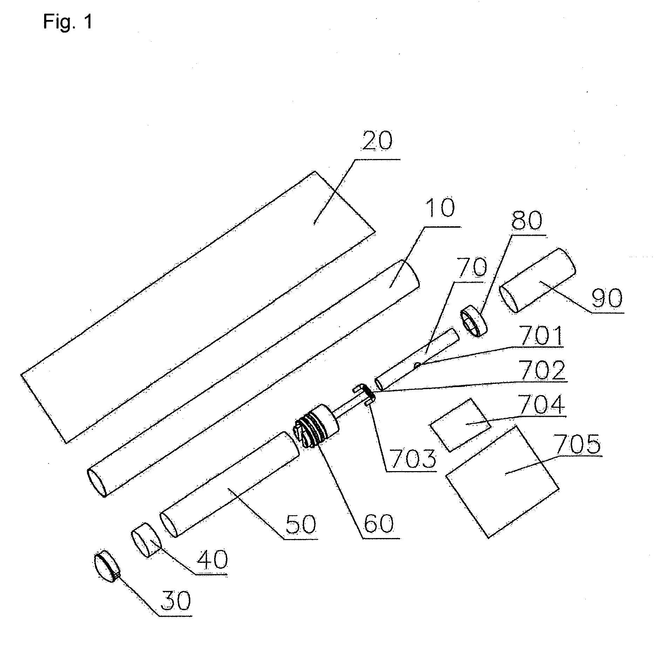

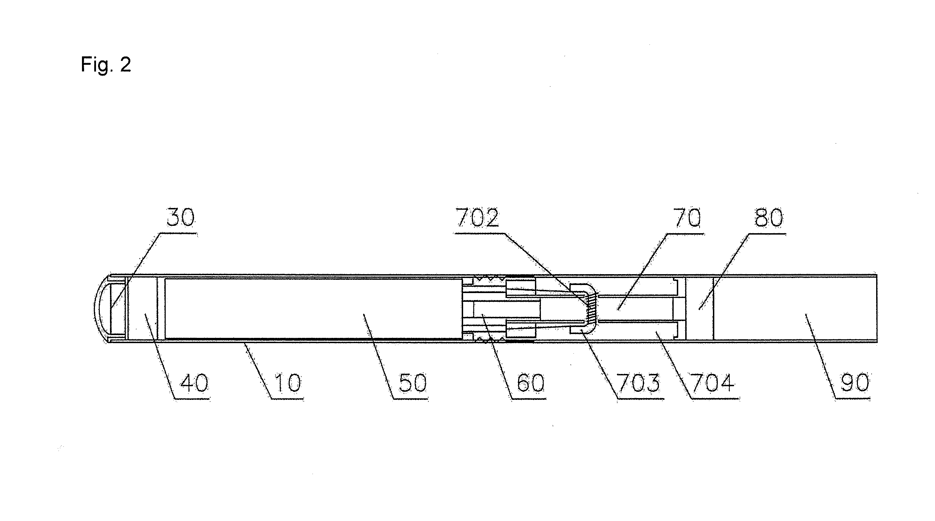

As shown in Figure 1 and Figure 2, this invention relates to an improved electronic cigarette structure, said electronic cigarette including a round tubular cigarette tube 10, the cigarette tube 10 being surrounded by a layer of adhesive paper 20, the length and diameter and design of said tubular pipe 10 being such that it simulates a real cigarette. The colour of said adhesive paper 20 also simulates a real cigarette, thus providing the best simulation. The front end of the cigarette tube 10 possesses a lamp cover 30. The lamp cover is equipped internally with an indicator light (not visible in the Figure) within said lamp cover 30. The end of tubular pipe 10 is equipped with a control board module 40, a battery 50, an atomizer base 60, a fibre glass tube 70 and a sealing ring 80, the indicator light within the lamp cover 30 being connected to the control board module 40 and the battery 50. When smoking, the control board module 40 can control the indicator light, simulating the cigarette burning and indicating that the electronic cigarette is working. When not smoking, the indicator is not lit. Diametrical hole 701 penetrates said fibreglass tube 70, hole 701 being internally equipped with fibreglass rope 703 wrapped in heating wire 702, the heating wire 702 being connected to the atomizer base 60. The hole 701 is surrounded by a layer of non-woven cotton fabric 704, the non-woven cotton fabric 704 being surrounded by a layer of oil-absorbing cotton 705.

The aforementioned sealing ring 80 is in a position at one end of the fibre glass tube 70, whilst the size of the hole in the sealing ring 80 is slightly smaller than the internal diameter of the glass tube 70. The sealing ring 80 forms a seal with the inner wall of the cigarette tube 10, ensuring that the smoke from the electronic cigarette is inhaled into the smoker's mouth through the cavities in the centre of the fibre glass tube 70 and centre of the sealing ring 80. In this embodiment, a mouthpiece structure is located at the rear end of the cigarette tube 10, said mouthpiece being of acetate fibre filter cotton 90. The acetate fibre filter cotton mouthpiece 90 is located on the outer side of the sealing ring 80. As such, the smoke sucked out of the aperture in the sealing ring 80 needs to pass through the acetate fibre filter cotton mouthpiece 90 before it can be inhaled into the smoker's mouth. This effectively prevents tar being sucked through the hole in the mouthpiece into the mouth of the smoker when the smoker applies excessive force or there is excessive tar. Apart from this, smokers who habitually bite cigarettes find the hard mouthpieces strange when they bite forcefully on the mouthpiece of traditional electronic cigarettes, whilst when smokers who habitually bite cigarettes bite forcefully on such rigid mouthpieces, this may result in the mouthpiece cover jumping into the smoker's mouth. The present invention uses an acetate fibre filter cotton mouthpiece 90, which not only makes the structure of the electronic cigarette more like a real cigarette, making the experience of smoking closer to that of smoking a real cigarette, but also prevents situations where the mouthpiece cover pops into the smoker's mouth, making usage safer and more reliable.

The above description is only a preferred embodiment of this invention and the above mentioned detailed implementation does not constitute any limitation to this invention. Various changes and modifications are permissible, and any refinement, modification or like-for-like exchange that may be made by a general technician in this field shall be covered by the scope of protection where these lie within the scope of the technical rationale of this invention.

## Claims

1. An electronic cigarette structure, including a round tubular cigarette tube and a control board module, a battery, an atomizer base, a fibreglass tube and a sealing ring installed within said tubular pipe from front to back, a diametrical hole penetrating that fibre glass tube, wherein the aforementioned hole is internally equipped with glass fibre rope around which heating wire is wrapped, the heating wire being connected to the atomizer base, there being a cigarette mouthpiece installed at the rear end of the cigarette tube on the outside of the sealing ring, said mouthpiece being an acetate fibre filter cotton mouthpiece.

2. The electronic cigarette structure according to claim 1, wherein the front end of the aforementioned cigarette tube is covered by a lamp cover, the lamp cover being equipped with an indicator light, said indicator light being connected to the control board module and battery and being controlled by the control board module.

3. The electronic cigarette structure according to claim 1, wherein the aforementioned hole in the fibreglass tube is surrounded by a layer of non-woven cotton fabric.

4. The electronic cigarette structure according to claim 3; wherein the aforementioned non-woven cotton fabric is surrounded by a layer of oil-absorbing cotton.

5. The electronic cigarette structure according to claim 1, wherein the aforementioned cigarette tube is surrounded by a layer of adhesive paper.
